# EUROPEAN PATENT APPLICATION

(11) **EP 4 024 885 A1**
(43) Date of publication of application: **06.07.2022**
(21) Application number: 20858854.1
(22) Date of filing: 11.09.2020
(51) Int. Cl.: H04R 1/10, A61B 5/055

(54) **NOISE REDUCTION CONVERSATION SYSTEM**

(30) Priority: 28.08.2019 CN 201910836398; 07.09.2019 CN 201910878554; 25.09.2019 CN 201910961238; 26.06.2020 CN 202010623416; 12.08.2020 CN 202010808736
(71) Applicant: Zhu, Aidao, Zhejiang 325002 (CN)
(72) Inventor: Zhu, Aidao, Zhejiang 325002 (CN)
(74) Representative: Hofstetter, Schurack & Partner
(86) International application number: PCT/CN2020/000219
(87) International publication number: WO 2021/036154

(57) **Abstract**

The present invention relates to a noise reduction communication system, which comprises a sending and receiving device arranged in a control room, an acoustic-electro conversion device arranged in a scanning room, and an air tube microphone headset connected to the aoustic-electro conversion device. The sending and receiving device is connected with the acoustic-electro conversion device. The air tube microphone headset is used for communication between the control room and the scanning room, wherein the noise reduction module is provided after the acoustic signals of the air tube microphone headset and the acoustic-electro conversion device are converted into electrical signals. The present invention can not only realize two-way wireless transmission of voice messages between the control room and the scanning room of MRI, but also reduce the noise during the transmission, thus improving the quality of voice transmission.

## Description

### Field of the Invention

The invention relates to a two-way communication device without a magnetic field or audio signals entering a special environment, in particular to a noise reduction communication system.

### Background of the Invention

The existing communication devices are generally suitable for conventional application scenarios. There are some special or extreme scenarios, such as one that has strong magnetic fields and strong noise and even together with weak electromagnetic fields where demands no active magnetic signals to interfere with them, for example, the above scenario with strong magnetic fields and strong noise that ordinary people may encounter is a scanning room where MRI scanner is located. So the present patent aims to solve this problem.

During the MRI examination, an examinee is in the scanning room, and an equipment operator the is in the control room to scan the examinee. But the scanning room and the control room are completely isolated by a shielding layer, which leads to a sound isolation between the control room and the scanning room, and lead to a difficult communication between the examinee and the operator.

Due to the strong magnetic field and huge noise produced by the MRI instrument, the current communication devices with metal materials are not suitable to be used, so the present solution is to install a speaker on the wall of the scanning room. However, in order to ensure the normal operation of the MRI instrument, the speaker is usually installed very high and far away from the inspected person, plus that the noise in the scanning room is very loud, which would lead to unclarity and even misdiagnosis.

In addition, because the speaker is generally unidirectional and can only transmit the voice from the control room to the scanning room, so the examinee in the scanning room can not actively communicate with the operator in the control room and can only act unilaterally under the operator's instructions. The bidirectional communication can not be made when the examinee has discomfort or emergency, thus, there would be false detection of MRI examination, and the accuracy and reliability would also be reduced, and even an interruption of the examination can be caused.

At the same time, some of the patients lie on the scanning bed without wearing a oxygen mask. It takes 20 minutes at least for them to be scanned and 40 minutes or more if multiple body parts are to be scanned, during which time, if the patients are uncomfortable and unable to tell the operator in the control room, there will be potential risks.

In addition, some of the patients are timid, and the sound is loud when they are pushed into the round hole with a large magnetic field. So the patients tend to feel panic and frightened.

In order to realize the communication between the MRI scanning room and the control room, some manufacturers have set up an alarm airbag for patients in the scanning room. Once the patients need to speak, they can trigger the alarm airbag by hand and tell the operator in the control room to turn off the MRI equipment and stop the scanning, then the patients can speak to the control room, after finishing it the operator in the control room need to restart the MRI equipment for scanning. It's very troublesome, time-consuming, and susceptible to quality problems in scanning.

### Summary of the Invention

In view of the shortcomings of the prior art, the present invention aims to provide a noise reduction communication system for MRI examination.

To achieve the above purpose, the present invention provides the following technical solution:
A noise reduction communication system comprises:
A sending and receiving device arranged in a control room;
An acoustic-electro conversion device arranged in a scanning room, and an air tube microphone headset connected to the acoustic-electro conversion device.

The sending and receiving device is connected with the acoustic-electro conversion device. The air tube microphone headset is used for communication between the control room and the scanning room, wherein the noise reduction module is provided after the acoustic signals of the air tube microphone headset and the acoustic-electro conversion device are converted into electrical signals.

The air tube microphone headset includes a headset frame which has sound output ports incorporated on both sides therein and a sound collector on either side. The sound output ports and the sound collectors are connected with the acoustic-electro conversion device through independent air tubes.

The two sound output ports are respectively connected with a third air tube through a first air tube and a second air tube. The sound collector is connected with a fifth air tube through a fourth air tube. The third air tube and fifth air tube are independent of each other and are connected with the acoustic-electro conversion device.

The middle of the air tube microphone headset is provided with an air tube socket.

A set of noise reduction modules comprise a first and second noise reduction module connected with a microphone for detecting ambient noise, a third and fourth noise reduction module connected with a speaker which is further linked to an air tube of a noise collecting head, and a noise reduction module connected with a microphone which is further linked to a sound collector of the air tube microphone headset through a connection of an air tube.

The sound collector of the air tube microphone headset is provided with a diaphragm which is connected with the microphone through the air tube. The microphone having an amplifier incorporated therein is connected with the noise reduction module via a metal shielded wire, and after spectrum SNR (signal-to-noise ratio) algorithm processing, the noise is filtered and transmitted to a sending and receiving device in the control room through a filter.

The audio signals from the sending and receiving device in the control room go through a filter and are transmitted to the speaker with an acoustic wave concentration port via a conducting line. Before transmitting to the speaker, the audio signals are processed by an AC signal with an anti-phase waveform sent by the noise reduction module to cancel out the noise received by patients.

The vibration sound collected by a noise collecting head and then generated by the diaphragm is transmitted to the microphone through the air tube. The air tube transmits audio signals generated by the microphone to the noise reduction modules to reduce noise where anti-phase signals are generated and transmitted to the speaker.

The microphone collects ambient noise and performs feedforward active noise reduction through the noise reduction modules to produce anti-phase signals which are to be transmitted to the speaker.

The sending and receiving device is connected to the acoustic-electro conversion device in a wireless manner.

The sending and receiving device comprises a microphone, a control machine and a control room transceiver which are connected sequentially. The antenna made of radio frequency cable of the control room transceiver is installed in the scanning room near the shielding wall through a filter. The acoustic-electro conversion device is connected with a patient transceiver, and the transceiver is provided with a patient transceiver antenna. The antenna of the control room transceiver and the antenna of the patient transceiver form a communication connection.

The control room transceiver antenna and patient transceiver antenna are directional antennas and the patient transceiver antenna's transmission power is lower than the one of the control room transceiver antenna.

The sending and receiving device is connected to the acoustic-electro conversion device in a wired manner.

The sending and receiving device comprises a first receiving channel and a first sending channel; the acoustic-electro conversion device comprises an independent second receiving channel and an independent second sending channel. The first receiving channel and the second sending channel, and the first sending channel and the second receiving channel are respectively connected by air tubes.

The first receiving channel comprises a microphone with an amplifier, an amplifying circuit and a speaker which are connected sequencially.

The first sending channel comprises a speaker with an acoustic wave concentrator, an amplifying circuit and a microphone, which are connected sequencially.

The second receiving channel comprises a shielding box where a speaker with an acoustic wave concentrator, an amplifying circuit and a microphone with an amplifier are connected sequentially. The microphone is connected with the first sending channel through an air tube, the speaker is connected with a jack of the socket through an air tube.

The second sending channel comprises a shielding box, where a speaker with an acoustic wave concentrator, an amplifying circuit and a microphone with an amplifier are connected sequentially. The speaker is connected with the first receiving channel through an air tube, and the microphone is connected with a jack of the socket through an air tube .

The control room transceiver comprises a control room transceiver integrated service processing unit, a control room transceiver analog-to-digital conversion unit, a control room transceiver digital-to-analog conversion unit, a control room transceiver zero-intermediate frequency conversion part unit, a control room transceiver limiting filter unit, a control room transceiver power amplifier unit, a control room transceiver switch and a control room transceiver antenna.

The patient transceiver comprises a patient transceiver integrated service processing unit, a patient transceiver analog-to-digital conversion unit, a patient transceiver digital-to-analog conversion unit, a patient transceiver zero-intermediate frequency conversion part unit, a patient transceiver limiting filter unit, a patient transceiver power amplifier unit, a patient transceiver switch and a patient transceiver antenna.

The audio signals transmitted by the sending and receiving device are successively processed by the control room transceiver integrated service processing unit, the control room transceiver analog-to-digital conversion unit, the control room transceiver zero-intermediate frequency conversion part unit, the control room transceiver power amplifier unit, the control room transceiver switch and the control room transceiver antenna and then converted into wireless signals. The wireless signals are directionally transmitted from the control room transceiver antenna to the patient transceiver antenna and processed successively by the patient transceiver switch, the patient transceiver limiting filter unit, and the zero-intermediate frequency conversion part unit of the patient transceiver, the analog-to-digital conversion unit and the integrated service processing unit of the patient transceiver. Finally, the wireless signals are converted into audio signals for transmission to the acoustic-electro conversion device.

The audio signals transmitted by the acoustic-electro conversion device are transformed into wireless signals after being processed by the patient transceiver integrated service processing unit, the patient transceiver digital-to-analog conversion unit, the patient transceiver zero-intermediate frequency conversion part unit, the patient transceiver power amplifier unit, the patient transceiver switch and the patient transceiver antenna. The wireless signals are directionally transmitted from the patient transceiver antenna to the control room transceiver antenna and then successively processed by the control room transceiver switch, the control room transceiver limiting filter unit, the control room transceiver zero-intermediate frequency conversion part unit, the control room transceiver analog-to-digital conversion unit and the control room integrated service processing unit. Finally, the wireless signals are converted into audio signals for transmission to the sending and receiving device.

Both the patient transceiver and the control room transceiver are provided with an antenna that can be configured and modulated for transmitting circuit. The antenna is for a directional narrow transmission channel. A narrow channel is provided between the patient transceiver and the control room transceiver and is used for signal transmission and exchange of the antenna.

The narrow channel is located outside the scanning area.

The patient transceiver is provided with a wireless transmitting airbag alarm device which has an airbag ball incorporated therein. The patient transceiver is further provided with a pneumatic switch and a control alarm circuit, wherein one end of the control alarm circuit is connected with the pneumatic switch and the other end of the pneumatic switch is connected with one end of an air tube through a connector, the other end of the air tube is connected with the airbag ball, and the other end of the control alarm circuit is connected with the integrated service processing unit of the patient transceiver.

The second sending channel comprises a shielding box and a shielding box, wherein the shielding box is provided with a microphone with an amplifier, an amplifying circuit and a speaker with an acoustic wave concentrator which are successively connected, wherein a speaker is connected with an amplifying circuit through an air tube. A noise reduction module, an amplifying circuit and a speaker with an acoustic wave concentrator are successively connected in the shielding box, wherein the small end of the acoustic wave concentrator is connected with the small end of the amplifier through an air tube, and the other end of the noise reduction module is connected with the small microphone.

The first sending channel comprises a speaker and a integrated unit which are connected successively.

the second sending channel comprises a shielding box where a noise reduction module, an amplifying circuit and a speaker with an acoustic wave concentrator are successively incorporated therein. The small end of the acoustic wave concentrator is connected with the small end of an amplifier outside of the shielding box through an air tube. The big end of the amplifier is connected with a microphone, the other end of the noise reduction module is linked to a small microphone, and the other end of the microphone is connected with a filter.

The first sending channel comprises the speaker, a integrated unit and the filter which are connected successively.

A pneumatic sound producer is installed on the sound receiving surface of the sound collector in the air tube microphone headset and covered by a cover which is flexibly installed on the sound collector. The cover can be opened to remove the pneumatic sound producer and the airbag when the pneumatic sound producer is not needed.

If a patient needs to contact the doctor in the control room, press the airbag several times and the air pressure will be transmitted to the pneumatic sound producer through the passage of the air tube. The pneumatic sound producer will then give an alarm sound, which will be transmitted to the sound collector and the air tube microphone headset and then through the patient transceiver or the acoustic-electro conversion device and finally be delivered to the control room.

### The Advantages of the Present Invention

The air tube headset in the communication system of the present invention can be applied in the scenario with strong magnetic fields and noise. The audio signals transmitted by the sending and receiving device can be converted into the sound signals through the acoustic-electro conversion device, and then sound signals are transmitted to the ears of users through the lengthened air tube. The air tube headset does not contain metal materials, so it will not have linkage with the strong magnetic environment, especially suitable for MRI examination. The present invention can realize a two-way wireless transmission of voice messages between the MRI control room and scanning room. The more concise structure and longer voice transmission distance make the MRI equipment not be affected by the electromagnetic field of the audio signals from the voice call during the MRI scanning.

The acoustic-electro conversion device of the present invention is provided with a noise reduction module, which comprises an acoustic noise reduction device and a sound generating noise reduction device. The noise of the voice messages received in the control room and the scanning room is greatly reduced by setting up the acoustic noise reduction device and the sound generating noise reduction device, and the voice transmission quality is improved.

The antennas of the control room transceiver and the patient transceiver in the present invention are directional antennas. The transmitting power of the patient transceiver antenna is lower than that of the control room transceiver antenna. The present invention realizes the wireless transmission of voice messages between the control room and the scanning room through the control room transceiver and the patient transceiver, which makes the structure of the communication system more concise and the voice transmission distance longer.

### Brief Description of the Drawings

Fig. 1 is a block diagram of an embodiment of the present invention.
Fig. 2 is the structural diagram of the air tube microphone headset of the present invention.
Fig. 3 is a noise reduction schematic diagram of an embodiment of the present invention.
Fig. 4 is a block diagram of an embodiment (wireless connection) of the present invention.
Fig. 5 is a block diagram of an embodiment (wired connection) of the present invention.
Fig. 6 is a block diagram of an embodiment (noise reduction) of the present invention.
Fig. 7 is another block diagram of an embodiment (noise reduction) of the present invention.
Fig. 8 is a structural diagram of the communication system of the present invention.
Fig. 9 is a structural diagram of the communication system (with airbag) of the present invention.
Fig. 10 is a structural diagram of the airbag alarm device.
Fig. 11 is a block diagram of the directional transceiver unit.
Fig. 12 is a schematic diagram of the channel with the radio frequency of 2400mhz of the directional transceiver.
Fig. 13 is a schematic diagram of the antenna of the directional transceiver.
Fig. 14 is a structural diagram of the control room transceiver.
Fig. 15 is a structural diagram of the patient transceiver.
Fig. 16 is a structural diagram of the alarm airbag attached to the sound collector.

### Detail Description of the Preferred Embodiments

The technical solutions in the embodiments of the present invention will be described clearly and completely in combination with the drawings. Obviously, the described embodiments are only part of the embodiments of the present invention and based on which, all other embodiments obtained by the skilled person in the field without creative work belong to the protection scope of the present invention.

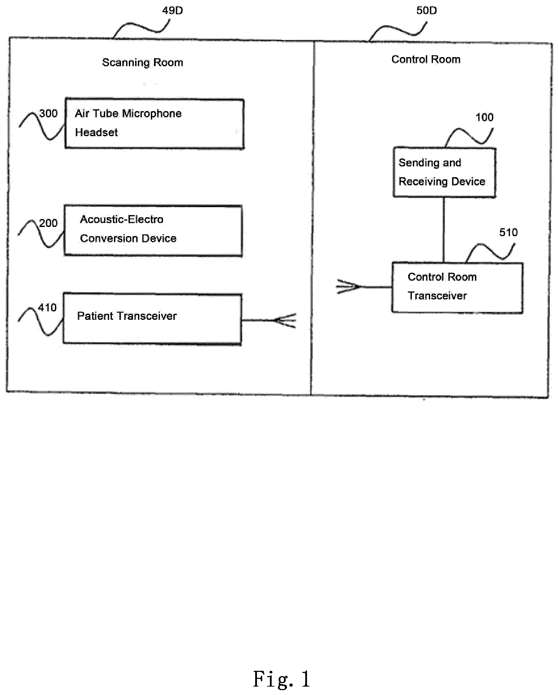

As shown in Fig. 1, a noise reduction communication system includes an air tube microphone headset 300 an acoustic-electro conversion device 200 a patient transceiver 410 in the scanning room 49D, and a sending and receiving device 100, a control room transceiver 510.in the control room 50D. The sound emitted by the patient is transmitted to the acoustic-electro conversion device 200 through the air tube microphone headset 300. The acoustic-electro conversion device 200 transmits the processed audio signals to the sending and receiving device 100 through the patient transceiver 410 and the control room transceiver 510.

The acoustic-electro conversion device 200 comprises a noise reduction module which is bidirectionally connected with the air tube microphone headset 300 and the patient transceiver 410 respectively.

The sending and receiving device 100 comprises a microphone, an amplifier circuit, a speaker and an acoustic wave concentrator, wherein the acoustic wave concentrator is connected with the acoustic-electro conversion device 200 through an air tube.

Specifically, when a person in the control room 50D needs to send voice messages to a person in the scanning room 49D, the sending and receiving device 100 collects the voice messages from the person in the control room 50D and converts them into audio signals; then the control room transceiver 510 receives the audio signals and converts them into wireless signals. The converted wireless signals are transmitted to the patient transceiver 410 through the wireless network and converted into audio signals and further into sound signals by the acoustic-electro conversion device 200 and then transmitted to the air tube microphone headset 300 through which the person in the scanning room 49D can receive the voice messages sent by the person in the control room 50D.

When a person in the scanning room 49D needs to send voice messages to a person in the control room 50D, the air tube headset 300 collects the sound signals formed by the voice messages from the person in the scanning room 49D and transmits them to the acoustic-electro conversion device 200 which further converts the sound signals into audio signals and transmits them to the patient transceiver 410; then the patient transceiver 410 converts the audio signals into wireless signals. The converted wireless signals are transmitted to the control room transceiver 510 through the wireless network and further converted into corresponding audio signals and then transmitted to the sending and receiving device 100 which converts the audio signals into corresponding sound signals and then broadcasts them to the person in the control room 50D.

The control room transceiver 510 and the patient transceiver 410 can replace or shorten the audio wire between the sending and receiving device 100 and the acoustic-electro conversion device 200 in above embodiments, which reduces the wiring of the communication system and makes the structure of the communication system more concise.

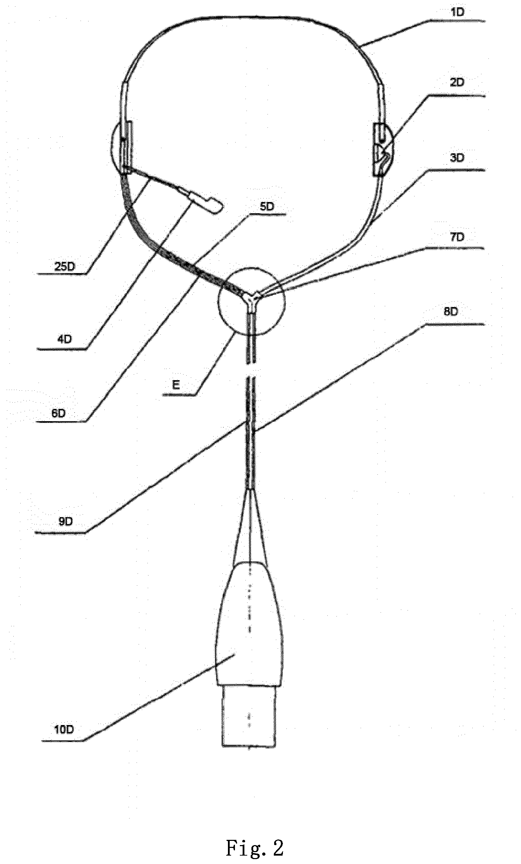

The air tube microphone headset as shown in Fig. 2. includes a headset frame 1D which has sound output ports 2D incorporated on both sides therein and sound collectors 4D on either side. The sound output ports 2D and the sound collector 4D are connected with the acoustic-electro conversion device through independent air tubes. The two sound output ports 2D are respectively connected with the third air tube 8D through the first air tube 5D and the second air tube 3D. The sound collector 4D is connected with the fifth air tube 9D through the air tube fourth 6D. The third air tube 8D and the fifth air tube 9D are independent of each other and are connected with the acoustic-electro conversion device. The sound collector 4D is provided with a diaphragm. The diaphragm is installed at the big end of the acoustic wave concentrator, of which the small end is connected with one end of the air tube 6D. The fourth air tube 6D is installed on a transmission arm 25D which can be freely bent and adjusted. The transmission arm 25D is made of plastic, elastic copper or 318/ 316L stainless steel.

The middle of the air tube microphone headset is provided with an air tube socket 10D which can be fixed by the plug-in method and the air tube microphone headset can be replaced.

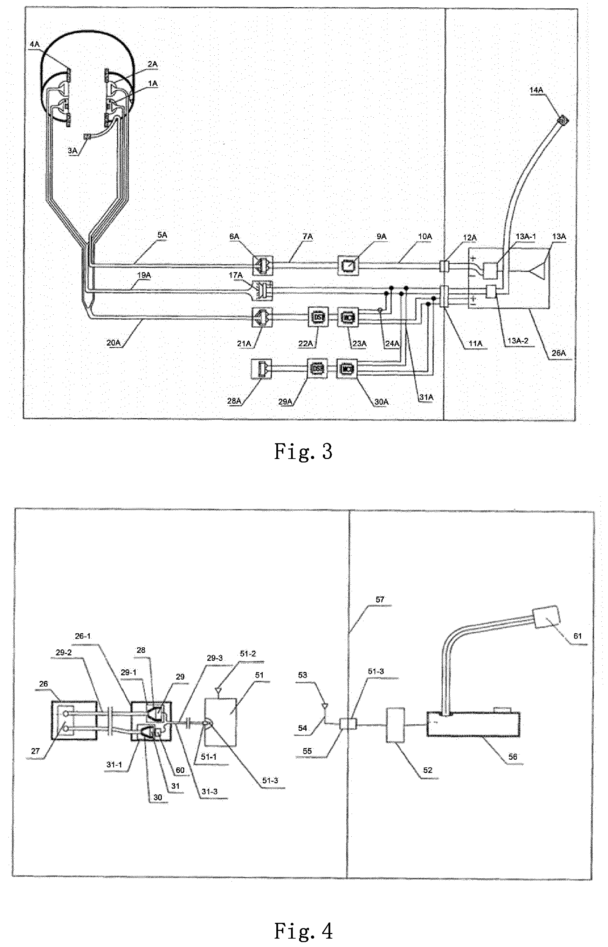

As is shown in Fig.3, a set of noise reduction modules comprise a noise reduction module 29A connected with a microphone 28A for detecting ambient noise, a noise reduction module 30A, a noise reduction module 22A and a noise reduction module 23A connected with a speaker 17A which is further linked to an air tube of a noise collecting head 2A, a noise reduction module 9A connected with a microphone 6A which is further linked to a sound collector of the air tube microphone headset through a connection of an air tube.

The sound collector of the air tube microphone headset is provided with a diaphragm. The diaphragm is connected with the microphone 6A which has an amplifier incorporated through the air tube. The microphone 6A is connected with the noise reduction module 9A through a metal shielded wire 7A, and after spectrum SNR (signal-to-noise ratio) algorithm processing, the noise is filtered and transmitted to a integrated unit 26A in the control room through a filter 12A, after which the audio signals are output through a speaker .

The audio signals from the integrated unit 26A in the control room go through the filter 11A and then the noise reduction module 23A which send the AC signals of anti-phase waveform to cancel out the noise patients receive, after which the signals are transmitted to the speaker 17A with an acoustic wave concentration port via a wire.

The vibration sound generated by the diaphragm and collected by the noise collecting head 2A is sent to the microphone 21A through an air tube. The audio signals generated by the microphone 21A are sent to the noise reduction module 22A and the noise reduction module 23A for noise reduction, during which the anti-phase signals which are to be transmitted to the speaker 17A are generated. To shorten the transmission time, the microphone 21A can also be used obtain noise .

The microphone 28A collects ambient noise and performs feedforward active noise reduction through the noise reduction module 29A and the noise reduction module 30A to produce inverse phase signals which are to be transmitted to the speaker 17A.

The sound generating and noise reduction process of patients is as follows:
The patient sound collector 3A is provided with a diaphragm and connected to the microphone 6A with an amplifying port through the tube 5A of the air tube microphone 1A to form the air tube microphone. The audio signals of the air tube microphone are sent to the patient voice-sending noise reduction module 9A through the metal shielded wire 7A for spectrum signal-to-noise ratio calculation processing, through which the noise is filtered out, and the voice is sent to the amplifying circuit 13A-1 set in the control room 26A through a half indoor-half outdoor filter 12A for tuning processing, then the voice will be broadcast by the speaker 13A to the person in the control room.

The power is provided by a regulated power supply or charged by a charger.

Music can be played in the communication channel between the control room and the scanning room and it can also be turned off or adjusted at any time.

The sound receiving and noise reduction process of patients is as follows:
The audio signals of microphone 14A pass through a half indoor-half outdoor filter 11A, and the noise patients receive is canceled out by the AC signals of anti-phase waveform before transmitted to the speaker 17A with an acoustic wave concentration port via a wire for sound production. The noise comes from the vibration sound generated by the diaphragm and collected by the noise collecting head and is sent to the microphone 21A through the air tube 20A. The audio signals generated by the microphone 21A are sent to the noise reduction module 22A and the noise reduction module 23A for noise reduction, during which process the anti-phase signals 27A which are to be transmitted to the speaker 17A via wire 24A are generated to cancel out the noise signals, achieving the effect of noise reduction.

The ambient noise reduction process is as follows:
The microphone 28A collects ambient noise and performs feedforward active noise reduction through the noise reduction module 30A to produce anti-phase signals which are to be transmitted to the speaker 17A to cancel out ambient noise.

As shown in Fig. 4, the sending and receiving device comprises a microphone, a control machine 56 and a control room transceiver 52 which are connected sequentially. The antenna 53 of the control room transceiver 52 is installed in a scanning room near the shielding wall through a filter 51-3 with a radio frequency cable 54. The acoustic-electro conversion device is connected with a patient transceiver 51-1, and the transceiver 51-1 is provided with a patient transceiver antenna 51-2. The antenna of the control room transceiver and the patient transceiver antenna form a communication connection.

The antenna 53 of the control room transceiver and the patient transceiver antenna 51-2 are directional antennas, and the transmission power of the patient transceiver antenna 51-2 is lower than that of the antenna 53 of the control room transceiver.

The jack 27 in the socket 26 is connected with the air tube socket 10D of the air tube microphone headset. One of the two small holes in the jack 27 is connected with the small end of the acoustic wave concentrator 28 via an air tube 29-2, and the big end of the acoustic wave concentrator 28 is provided with the speaker 29, of which the wire 29-3 is connected with a plug 51-1.The plug 51-1 is connected with the socket 51-3 of the patient transceiver 51.

The other small hole of the jack 27 is connected to the amplifying port 30 via an air tube 31-2. The big end of the amplifying port 30 is provided with a microphone 31 which is connected with a noise reduction module 60. The noise reduction module 60 is connected with another wire of plug 51-1 via the wire 31-3, and the plug 51-1 is connected with the socket 51-3 of the patient transceiver 51.

The antenna of the patient transceiver 51-2 exchanges signals with the antenna of the control room transceiver 53 in a wireless manner. The antenna of the control room transceiver 53 is connected with the control room transceiver 52 via the RF cable 54 and the filter 55. The received audio signals after signal processing is connected with the control machine 56 through the control room transceiver 52 via the wire or the wire with a plug.

The patient's transceiver 51 is installed outside the scanning area of MRI so as not to affect the scanning.

Bluetooth is chosen for the circuit of the patient transceiver 51, and the Bluetooth functional circuit and element for a mobile phone or the ones of Bluetooth are for the control room transceiver 52. The ones of Bluetooth requires lowered power of the antenna, shortened transmitting distance and selected transmitting and receiving frequency so as not to influence the MRI scanning. The output power should meet the needs.

The transmitting power of the antenna 51-2 of the patient transceiver is lower than that of the antenna 53 of the control room transceiver, which is set to achieve no influence on the MRI scanning. Since the control room transceiver 52 is far from the scanning area, a certain amount of transmitted signals can be attenuated so the quality of the MRI scanning will not be affected.

The patient transceiver 51 and the control room transceiver 52 are powered by chargers or regulated power supply.

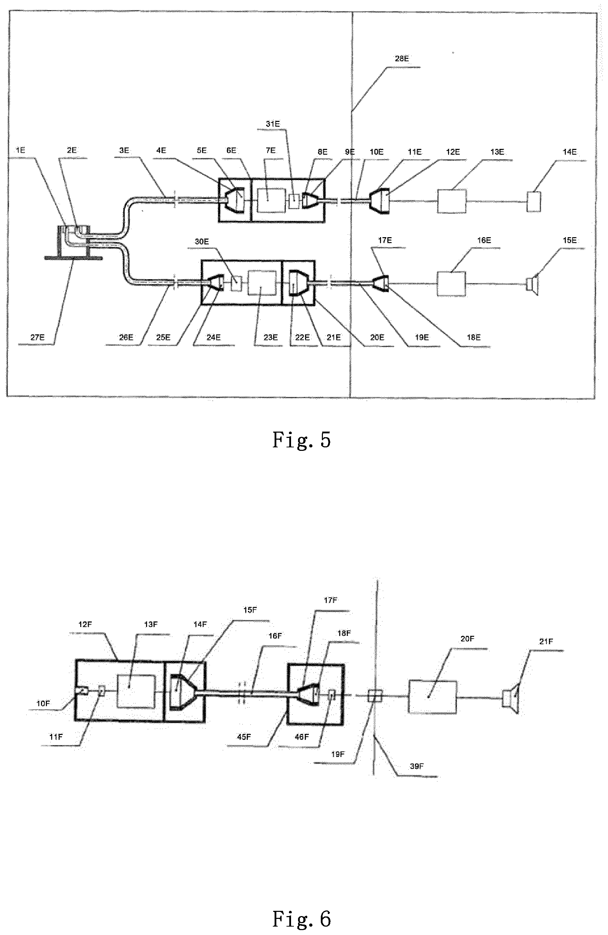

As shown in FIG. 5, a wired connection is made between the receiving device and the acoustic-electro conversion device.

The sending and receiving device comprises a first receiving channel and a first sending channel; the acoustic-electro conversion device comprises an independent second receiving channel and a second sending channel. The first receiving channel and the second sending channel, and the first sending channel and the second receiving channel are respectively connected by air tubes.

The first receiving channel comprises a microphone 18E with an amplifier 17E, an amplifying circuit 16E and a speaker 15E which are connected sequentially.

The first sending channel comprises a speaker 12E with an acoustic wave concentrator 11E, an amplifying circuit 13E and a microphone 14E which are connected one after another.

The second receiving channel comprises a shielding box 6E where a speaker 5E with an acoustic wave concentrator 4E, an amplifying circuit 7E and a microphone 8E with an amplifier 9E are successively connected, wherein the microphone 8E is connected with the first sending channel via an air tube 10E and the speaker 5E is connected with a jack 2E of the socket 27E via an air tube 3E.

The second sending channel comprises a shielding box 20E where a speaker 22E with an acoustic wave concentrator 21E, an amplifying circuit 23E and a small microphone 24E with an amplifier 25E, are successively connected, wherein the speaker 22E is connected with the first receiving channel via an air tube 19E and the microphone 25E is connected with a jack 1E of the socket 27E via an air tube 26E.

The sound signals from the speaker 12E in the control room are sent to the microphone 8E which is in the shielding box 6E by the acoustic wave concentrator 11E through the air tube 10E. An amplifying port 9E is set between the air tube 10E and the microphone 8E. The microphone 8E generates electrical signals which are amplified by the amplifying circuit 7E and then the signals are sent to the speaker 5E to produce sound. The sound is sent to the socket 27E through the acoustic wave concentrator 4E and the air tube 3E, and further delivered to the patient via the air tube headset. The amplifying circuit and the speaker are both installed in the shielding box 6E.

The signals from the socket 27E of the air tube headset are transmitted to the small microphone 24E through the air tube 26E and then through the amplifying port 25E. The small microphone 24E sends the electrical signals to the amplifying circuit 23E for amplifying and then to the speaker 22E for sound production. The sound from the speaker 22E is further sent to the small microphone 17E in the main unit of the control room through the acoustic wave concentrator 21E and then the air tube 19E. The sound from the small microphone 17E is amplified by the amplifying circuit 16E and then sent to the speaker 15E for the person in the control room to listen.

The amplifying circuit in the scanning room is powered by a battery, which makes the sound both in the control room and the shielding room be amplified again, and the amplification function is improved. The air tube can be set smaller.

The transmitting circuits of transceiver 52E in control room G and transceiver 56E in scanning and shielding room F are modulated and the antennas 53E and 55E are respectively configured into a narrow and single-directional transmitting channel 51E with a certain width for transmitting and exchanging signals. The narrow channel 51E is located in the shielding room F but outside the scanning area 58E in the scanning room so as not to affect the MRI scanning quality.

The existing transmitting and receiving technology that can prevent the leakage of signals with high confidentiality is applied to the radio frequency circuit and antenna which are used as transceivers in the present patent, and do not affect the surrounding electromagnetic signals or the work of other active equipment.

Signals are transmitted between the transceiver 56E and the patient through the air tube microphone headset 59E, which ensures the passive and non-magnetic state in the scanning area and the quality of scanning.

The sound signals from the speaker 12E in the control room are sent to the microphone 8E in the shielding box 6E through the acoustic wave concentrator 11E and the air tube 10E. The amplifying port 9E is set between the air tube 10E and the microphone 8E; and the noise reduction module 31E is set between the microphone 8E and the amplifying circuit 7E. The microphone 8E generates electrical signals which pass through the noise reduction module 31E for noise reduction and then are amplified by the amplifying circuit 7E and sent to the speaker 5E to produce sound. The sound is sent to the socket 27E through the acoustic wave concentrator 4E and the air tube 3E, and further delivered to the patient via the air tube headset. The amplifying circuit and the speaker are both installed in the shielding box 6E.

The signals from the socket 27E are transmitted to the small microphone 24E through the air tube 26E and then through the amplifying port 25E. The small microphone 24E sends the electrical signals to the noise reduction module 30E for noise reduction, and then to the amplifying circuit 23E for amplifying and then sent to the speaker 22E for sound production. The sound of the speaker 22E is further sent to the small microphone 18E fixed on the big end of the amplifying port 17E in the main unit of the control room through the acoustic wave concentrator 21E and the air tube 19E. The sound from the small microphone 18E is amplified by the amplifying circuit 16E and then sent to the speaker 15E for the person in the control room to listen.

The amplifying circuit in the scanning room is powered by a battery, which makes the sound both in the control room and the shielding room be amplified again, and the amplification function is improved. The air tube can be set smaller.

As is shown in Fig.6, the second sending channel comprises a shielding box 12F which has a noise reduction module 11F, an amplifying circuit 13F and a speaker 14F with an acoustic wave concentrator 15F successively incorporated therein. The small end of the acoustic wave concentrator 15F is connected with the small end of an amplifier 17F located on the outside of the shielding box 12F through an air tube 16F. The big end of the amplifier port 17F is connected with a microphone 18F, the other end of the noise reduction module 11F is linked to a small microphone 10F, and the other end of the microphone 18F is connected with a filter 19F. The amplifier port 17F and the microphone 18F which is connected with a noise reduction module 46F are put together in a shielding cover 45F and the noise reduction module 46F is set outside the shielding cover 45F, which will have a better effect of noise reduction.

The first sending channel comprises the speaker 21F, a integrated unit 20F and the filter 19F which are connected successively.

The small microphone 10F is connected to the noise reduction module 11F which is linked to the amplifying circuit 13F via a wire for electrical signals amplification. The amplifying circuit 13F is connected to the speaker 14F which is installed at the big end of the acoustic wave concentrator 15F, of which the small end is linked to one end of the air tube 16F. The other end of the air tube 16F is connected to the acoustic wave amplifier 17F, of which the big end is provided with the microphone 18F. The microphone 18F joins the filter 19F via a wire. The other end of the filter 19F is linked to the integrated unit 20F in the control room where the sound will be amplified and then sent to the person in the control room through the speaker 21F.

The speaker 21F is installed in the integrated unit 20F in the control room.

The receiving device and the amplifying circuit in the shielding room both pass through an air tube, which isolates them from the control room, so the scanning quality will not be affected by external signals.

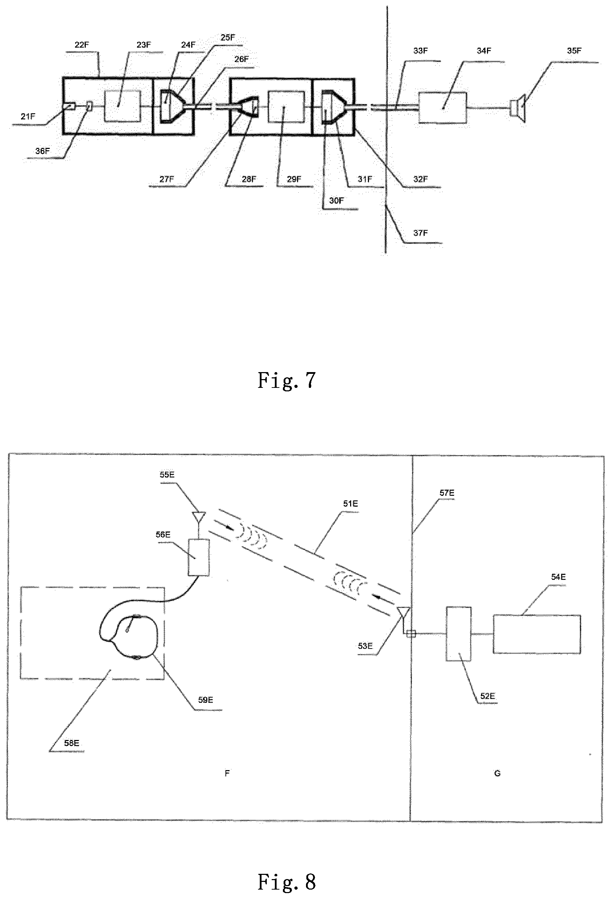

As is shown in Fig.7, the second sending channel comprises a shielding box 22F and a shielding box 32F, wherein the shielding box 32F is provided with a microphone 28F having an amplifier 27F, an amplifying circuit 29F and a speaker 30F with an acoustic wave concentrator 31F which are successively connected, wherein a speaker 30F is connected with a integrated unit 34F through an air tube 33F. And a noise reduction module 36F, an amplifying circuit 23F and a speaker 24F with an acoustic wave concentrator 25F are successively connected in the shielding box 22F, wherein the small end of the acoustic wave concentrator 25F is connected with the small end of the amplifier 27F through an air tube, and the other end of the noise reduction module 36F is connected with the small microphone 21F.

The first sending channel comprises a speaker 35F and a integrated unit 34F which are connected successively.

The staff in the scanning and shielding room are isolated by a two-level transmission device.

The small microphone 21F is connected to the noise reduction module 36F which is linked to the amplifying circuit 23F. The amplifying circuit 23F is connected to the speaker 24F which is installed at the big end of the acoustic wave concentrator 25F, of which the small end is linked to the air tube 26F. The other end of the air tube 26F is connected to the acoustic wave amplifier 27F, of which the big end is provided with the microphone 28F. The microphone 28F joins to the amplifying circuit 29F via a wire and the amplifying circuit 29F is connected to the speaker 30F via a wire. The speaker 30F is installed at the big end of the acoustic wave concentrator 31F, of which the small end is linked to the air tube 33F. The other end of the air tube 33F passes through the shielding wall 37F and joins the integrated unit 34F in the control room where sound will be amplified and then sent to the person in the control room through the speaker 35F.

At the same time, by setting a small microphone, the hands-free effect can be achieved.

As is shown in Fig.8, the transmitting circuits of transceiver 52E in control room G and transceiver 56E in scanning and shielding room F are modulated and the antennas 53E and 55E are respectively configured into a narrow and single-directional transmitting channel 51E with a certain width for transmitting and exchanging signals. The narrow channel 51E is located in the shielding room F and outside the scanning area 58E in the scanning room so as not to affect the MRI scanning quality.

The existing transmitting and receiving technology that can prevent the leakage of signals with high confidentiality is applied to the radio frequency circuit and antenna which are used as transceivers in the present patent, and do not affect the surrounding electromagnetic signals or the work of other active equipment.

Signals are transmitted between the transceiver 56E and the patient through the air tube microphone headset 59E, which ensures the passive and non-magnetic state in the scanning area and the quality of scanning.

The directional transmitting and receiving technology that can prevent the leakage of signals with high confidentiality is applied to the radio frequency circuit and antenna which are used as transceivers in the present patent, and do not affect the surrounding electromagnetic signals or the work of other active equipment.

An acoustic wave conversion device is arranged in the patient transceiver, and the air tube microphone headset 59E is connected with the patient transceiver by the air tube.

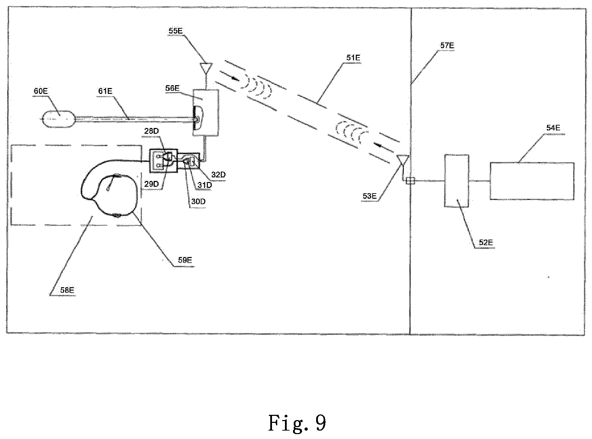

As shown in Fig. 9, an airbag 60E is added on the base of the arrangement in fig. 8. The airbag 60E is connected with the patient transceiver 56E through the air tube 61E, and the pressed alarm signals are sent through the antenna of the patient transceiver.

One end of the air tube microphone headset 59E is connected to the small end of the acoustic wave concentrator 28D via the air tube, and the big end of the acoustic wave concentrator 28D is provided with the speaker 29D, of which the wire is connected with the patient transceiver 56E.

The other end of the air tube microphone headset 59E is connected to the amplifying port 30D via the air tube, and the big end of the amplifying port 30D is provided with the microphone 31D, of which the wire is connected with the noise reduction module 32D, and the other end of the noise reduction module 32D is connected with the patient transceiver 56E.

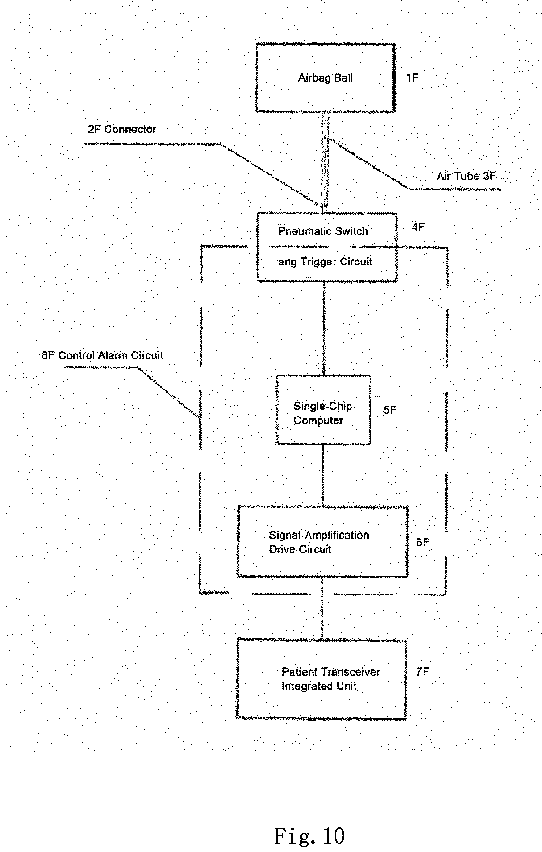

As shown in Fig. 10, the patient transceiver is provided with a wireless airbag alarm device which has an airbag ball 1F, a pneumatic switch 4F and a control alarm circuit 8F incorporated therein. One end of the control alarm circuit 8F is connected with the pneumatic switch 4F, of which the other end is connected with the air tube through the connector 2F. The other end of the air tube is connected with the airbag ball, and the other end of the control alarm circuit is connected with the integrated unit of the patient transceiver.

One end of the control alarm circuit 8F is connected with the pneumatic switch 4F, of which the other end is connected with the air tube 3F through the air tube connector 2F. The other end of the air tube 3F is connected with the airbag ball 1F, and the other end of the control alarm circuit 8F is connected with the integrated unit of the patient transceiver.

The control alarm circuit 8F is provided with a single-chip computer 5F and a signal-amplification drive circuit 6F, wherein the single-chip computer 5F is connected with the signal-amplification drive circuit 6F.

The alarm signals generated by the single-chip computer 5F consecutively pass through the integrated unit 7F of the patient transceiver, a baseband processing component and directional antenna of a front-end component of the transceiver to exchange signals with the directional antenna 518 of the transceiver in the control room. The alarm sound will be sent out after being processed by the transceiver in the control room.

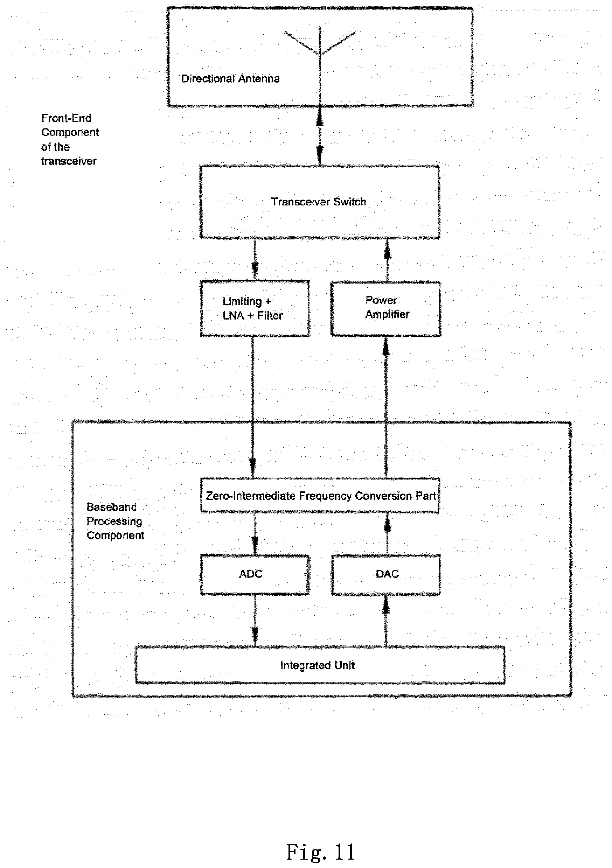

The structural diagram of a directional transceiver in a communication system is shown in Fig. 11. The control room transceiver (such as the control room transceiver 510 or 52E in the above embodiments) and the patient transceiver (such as the patient transceiver 410 or 56E in the above embodiments) are both directional transceivers with same structures. The directional transceiver performs as follows: the signals processed by the integrated unit go through a DAC unit, a zero-intermediate frequency conversion part, a power amplifier and a transceiver switch to the antenna for directional transmission. The process of receiving signals is as follows: the antenna receives the signals from the directional channel, and then successively goes through the transceiver switch, a limiting + LAN + filter, the zero-intermediate frequency conversion part in the baseband processing component and ADC to the integrated unit.

The main specifications of the high frequency part of the 2400mhz directional transceiver are as follows:
1) Working frequency: 2400MHz-2485MHz;
2) Input signal level: -107 ~ -16.5dBm;
3) Receiver noise factor: ≤ 4.0dB;
4) Rated output power of transmitter: 31.5dBm;
5) Antenna gain: ≥ 9dBi;
6) Beam width: 30 ports (typical value);
7) The communication distance is 10 meters.

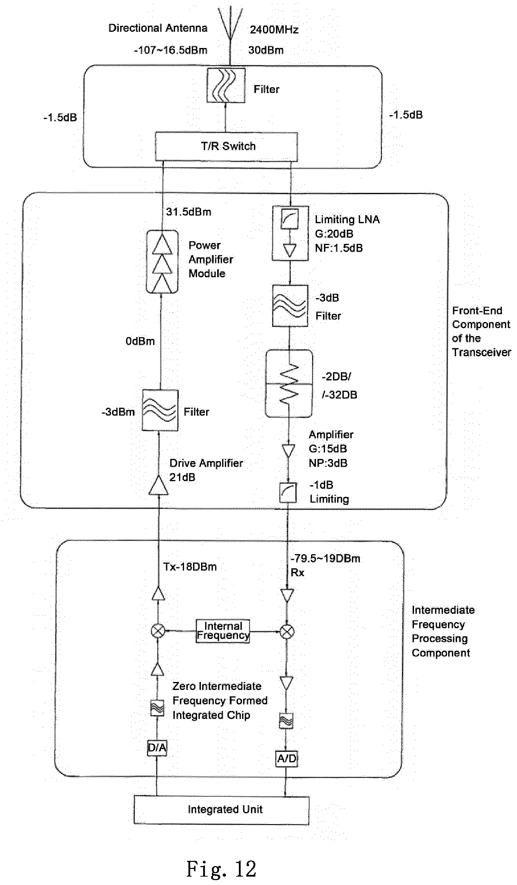

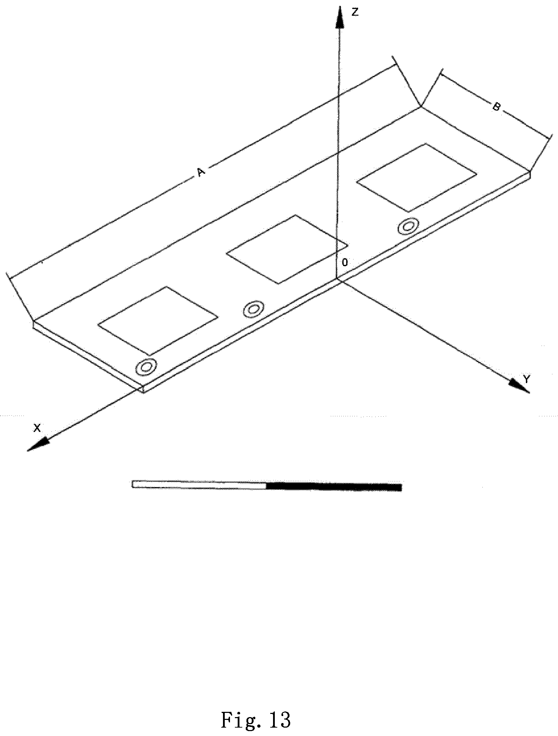

The design of the transceiver channel is shown in Fig. 12. The width of the RF channel of the transceiver is within ±15 ° margin of error. The antenna is about 190mm long and 60mm wide. The shape is shown in FIG. 13.

Both the control room transceiver antenna 418 and the patient transceiver antenna 518 are directional antennas with a length of 190mm and a width of 60mm. The directional antenna does not affect the surrounding electromagnetic signals or the work of other active equipment.

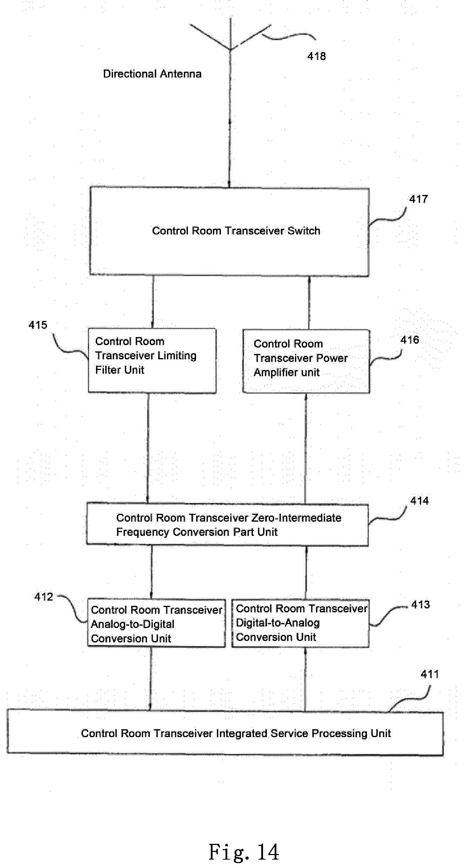

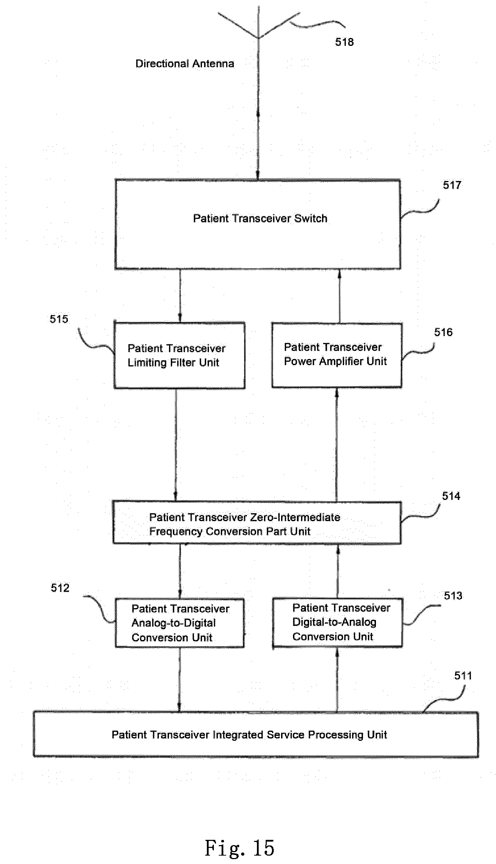

As shown in fig. 14 and fig. 15, the control room transceiver 52 comprises a control room transceiver integrated service processing unit 411, a control room transceiver analog-to-digital conversion unit 412, a control room transceiver digital-to-analog conversion unit 413, a control room transceiver zero-intermediate frequency conversion part unit 414, a control room transceiver limiting filter unit 415, a control room transceiver power amplifier unit 416, a control room transceiver switch 417 and a control room transceiver antenna 418.

The patient transceiver 51-1 comprises a patient transceiver integrated service processing unit 511, a patient transceiver analog-to-digital conversion unit 512, a patient transceiver digital-to-analog conversion unit 513, a patient transceiver zero-intermediate frequency conversion part unit 514, a patient transceiver limiting filter unit 515, a patient transceiver power amplifier unit 516, a patient transceiver switch 517 and a patient transceiver antenna 518.

The audio signals transmitted by the sending and receiving device are successively processed by the control room transceiver integrated service processing unit 411, the control room transceiver analog-to-digital conversion unit 412, the control room transceiver zero-intermediate frequency conversion part unit 414, the control room transceiver power amplifier unit 416, the control room transceiver switch 417 and the control room transceiver antenna 418 and then converted into wireless signals. The wireless signals directionally transmitted from the control room transceiver antenna 418 to the patient transceiver antenna 518 and successively processed by the patient transceiver switch 517, the patient transceiver limiting filter unit 515, and the patient transceiver zero-intermediate frequency conversion part unit 514, the patient transceiver digital-to-analog conversion unit 512 and the patient transceiver integrated service processing unit 511. Then the wireless signals are converted into audio signals for transmission to the acoustic-electro conversion device.

The audio signals transmitted by the acoustic-electro conversion device are transformed into wireless signals after being processed by the patient transceiver integrated service processing unit 511, the patient transceiver analog-to-digital conversion unit 513, the patient transceiver zero-intermediate frequency conversion part unit 514, the patient transceiver power amplifier unit 516, the patient transceiver switch 517 and the patient transceiver antenna 518. The wireless signals directionally transmitted from the patient transceiver antenna 518 to the control room transceiver antenna 418 and successively processed by the control room transceiver switch 417, the control room transceiver limiting filter unit 415, the control room transceiver zero-intermediate frequency conversion part unit 414, the control room digital-to-analog conversion unit 412 and the control room integrated service processing unit 411. Then the wireless signals are converted into audio signals for transmission to the sending and receiving device.

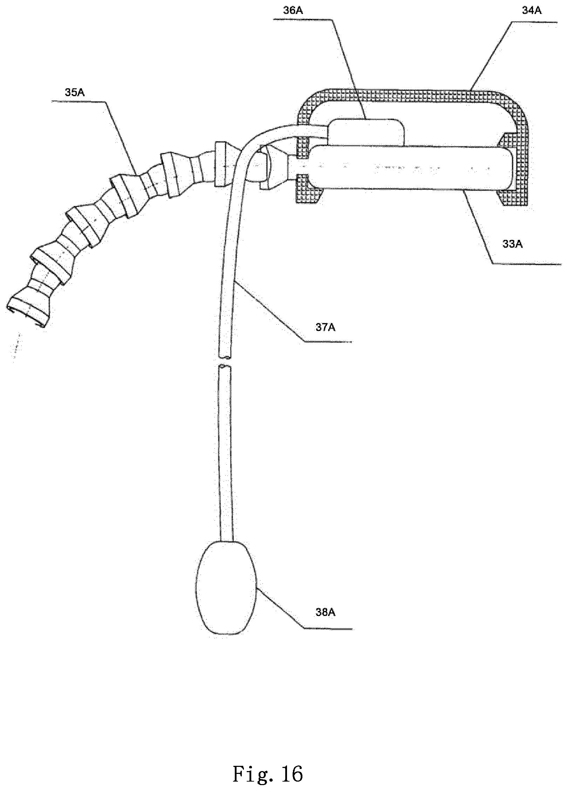

As is shown in Fig.16, the additional airbag alarm is provided for patients who can not speak. A pneumatic sound producer 36A is installed on the sound receiving surface of the sound collector 4D of the air tube microphone headset and covered by a cover 34A which is flexibly installed on the sound collector 4D. The cover 34A can be opened to remove the pneumatic sound producer 36A and the airbag 38A when the pneumatic sound producer 36A is not needed.

If a patient needs to contact the doctor in the control room, press the airbag 38A several times and the air pressure of the airbag 38A will be transmitted to the pneumatic sound producer 36A through the passage of the air tube 37A. The pneumatic sound producer 36A will then give an alarm sound, which will be transmitted to the sound collector 4D and the air tube microphone headset and then through the patient transceiver and finally be delivered to the control room.

Alternatively, the air tube microphone headset with the airbag alarm attached to its sound collector can be directly connected to the socket 27E in Figure 5 via the air tube socket 10D. The alarm signals can be transmitted to the control room through the acoustic-electro conversion device.

The embodiments shall not be regarded as a limitation of the present invention. And any improvement based on the spirit of the invention shall be within the protection scope of the present invention.

## Claims

1. A noise reduction communication system compromising:
A sending and receiving device (100) arranged in a control room;
An acoustic-electro conversion device (200) arranged in a scanning room, and an air tube microphone headset (300) connected to the acoustic-electro conversion device (200).
**characterized in**:
The sending and receiving device (100) is connected with the acoustic-electro conversion device (200). The air tube microphone headset is used for communication between the control room (50D) and the scanning room (49D), wherein a noise reduction module is provided after the acoustic signals of the air tube microphone headset and the acoustic-electro conversion device are converted into electrical signals.

2. The noise reduction communication system according to claim 1 wherein the air tube microphone headset includes a headset frame (1D) which has sound output ports (2D) incorporated on both sides and a sound collector (4D) on either side therein. The sound output ports (2D) and the sound collector (4D) are connected with the acoustic-electro conversion device by independent air tubes.

3. The noise reduction communication system according to claim 2 is **characterized in that** the two sound output ports are respectively connected with the third air tube (8D) by the first air tube (5D) and second air tube (3D). The sound collector (4D) is connected with the fifth air tube (9D) by the fourth air tube (6D). The third air tube (8D) and fifth air tube (9D) are independent of each other and are connected with the acoustic-electro conversion device .

4. The noise reduction communication system according to claim 1, 2 or 3 is **characterized in that** the middle of the air tube microphone headset is provided with an air tube socket (10D).

5. The noise reduction communication system according to claim 1, 2 or 3 wherein a set of noise reduction modules comprise a first noise reduction module (29A) and a second noise reduction module (30A) connected with a microphone (28A) for detecting ambient noise, a third noise reduction module (22A) and a fourth noise reduction module (23A)connected with a speaker (17A) which is further linked to an air tube(19A) of a noise collecting head(2A) , and a noise reduction module (9A) connected with a microphone (6A) which is further linked to a sound collector(3A) of the air tube microphone headset through a connection of an air tube(5A).

6. The noise reduction communication system according to claim 5 wherein the sound collector (3A) of the air tube microphone headset is provided with a diaphragm. The sound collector (3A) is connected with the microphone (6A) by the air tube (5A). The microphone (6A) with an amplifier incorporated therein is connected with the noise reduction module (9A) through a metal shielded wire (7A), and then after spectrum SNR (signal-to-noise ratio) algorithm processing, the noise is filtered and a filter (12A), of which one end is connected with the noise reduction module (9A), is linked to a integrated unit (26A) in the control room.

7. The noise reduction communication system according to claim 5 is **characterized in that** the audio signals from the integrated unit (26A) in the control room go through a filter (11A) and are canceled out by the AC signals of anti-phase waveform sent by the noise reduction module (23A) and then transmitted to the speaker (17A) equipped with an acoustic wave concentration port via a wire.

8. The noise reduction communication system according to claim 7 is **characterized in that** vibration sound generated by the diaphragm and collected by the noise collecting head (2A) is sent to the microphone (21A) through the air tube (20A). The audio signals generated by the microphone (21A) are sent to the noise reduction module (22A) and the noise reduction module (23A) for noise reduction, during which process the anti-phase signals which are to be transmitted to the speaker (17A) are generated.

9. A noise reduction communication system according to claim 5 is **characterized in that** the microphone (28A) collects ambient noise and performs feedforward active noise reduction through the noise reduction module (29A) and the noise reduction module (30A) to produce anti-phase signals which are to be transmitted to the speaker (17A).

10. The noise reduction communication system according to claim 1 is **characterized by** a wireless connection between the sending and receiving device and the acoustic-electro conversion device .

11. The noise reduction communication system according to claim 10 is **characterized in that** the sending and receiving device comprises a microphone (61), a control machine (56) and a control room transceiver (52) which are connected one after another. The antenna (53) of the control room transceiver (52) is installed in a scanning room near the shielding wall (57) through a filter (51-3) with a radio frequency cable (54). The acoustic-electro conversion device is connected with a patient transceiver (51-1), and the patient transceiver (51-1) is provided with a patient transceiver antenna (51-2). The antenna of the control room transceiver and the patient transceiver antenna form a communication connection.

12. The noise reduction communication system according to claim 11 is **characterized in that** the control room transceiver antenna (53) and the patient transceiver antenna (51-2) are directional antennas, and the transmission power of the patient transceiver antenna (51-2) is lower than that of the control room transceiver antenna (53).

13. The noise reduction communication system according to claim 1 is **characterized by** a wired connection between the sending and receiving device and the acoustic-electro conversion device.

14. The noise reduction communication system according to claim 13 is **characterized in that** the sending and receiving device comprises a first receiving channel and a first sending channel; the acoustic-electro conversion device comprises an independent second receiving channel and an independent second sending channel. The first receiving channel and the second sending channel, and the first sending channel and the second receiving channel are respectively connected by air tubes.

15. The noise reduction communication system according to claim 14 is **characterized in that** the first receiving channel comprises a microphone (18E) with a amplifier (17E), an amplifying circuit (16E) and a speaker (15E) which are connected one after another.
The first sending channel comprises a speaker (12E) with an acoustic wave concentrator (11E), an amplifying circuit (13E) and a microphone (14E) which are connected one after another.

16. The noise reduction communication system according to claim 14 is **characterized in that** the second receiving channel comprises a shielding box (6E) where a speaker (5E) with an acoustic wave concentrator (4E), an amplifying circuit (7E) and a microphone (8E) with an amplifier (9E) are successively connected, wherein the microphone (8E) is connected with the first sending channel through an air tube (10E), the speaker (5E) is connected with a jack (2E) of the socket (27E) through an air tube (3E).
The second sending channel comprises a shielding box (20E) where a speaker (22E) with an acoustic wave concentrator (21E), an amplifying circuit (23E) and a microphone (24E) with an amplifier (25E) are successively connected, wherein the speaker (22E) is connected with the first receiving channel through an air tube (19E), and the microphone (24E) is connected with a jack (1E) of the socket (27E) through an air tube (26E).

17. The noise reduction communication system according to claim 11 or 12 is **characterized in that** the control room transceiver (52) comprises a control room transceiver integrated service processing unit (411), a control room transceiver analog-to-digital conversion unit (412), a control room transceiver digital-to-analog conversion unit (413), a control room transceiver zero-intermediate frequency conversion part unit (414), a control room transceiver limiting filter unit (415), a control room transceiver power amplifier unit (416), a control room transceiver switch (417) and a control room transceiver antenna (418).
The patient transceiver (56E) comprises a patient transceiver integrated service processing unit (511), a patient transceiver analog-to-digital conversion unit (512), a patient transceiver digital-to-analog conversion unit (513), a patient transceiver zero-intermediate frequency conversion part unit (514), a patient transceiver limiting filter unit (515), a patient transceiver power amplifier unit (516), a patient transceiver switch (517) and a patient transceiver antenna (518).
The audio signals transmitted by the sending and receiving device are successively processed by the control room transceiver integrated service processing unit (411), the control room transceiver analog-to-digital conversion unit (412), the control room transceiver zero-intermediate frequency conversion part unit (414), the control room transceiver power amplifier unit (416), the control room transceiver switch (417) and the control room transceiver antenna (418) and then converted into wireless signals. The wireless signals are directionally transmitted from the control room transceiver antenna (418) to the patient transceiver antenna (518) and successively processed by the patient transceiver switch (517), the patient transceiver limiting filter unit (515), and the zero-intermediate frequency conversion part unit (514) of the patient transceiver, the patient transceiver digital-to-analog conversion unit (512) and the patient transceiver integrated service processing unit (511) to convert the wireless signals into audio signals for transmission to the acoustic-electro conversion device;
The audio signals transmitted by the acoustic-electro conversion device are transformed into wireless signals after being processed by the patient transceiver integrated service processing unit (511), the patient transceiver analog-to-digital conversion unit (513), the patient transceiver zero-intermediate frequency conversion part unit (514), the patient transceiver power amplifier unit (516), the patient transceiver switch (517) and the patient transceiver antenna (518). The wireless signals directionally transmitted from the patient transceiver antenna (518) to the control room transceiver antenna (418) and successively processed by the control room transceiver switch (417), the control room transceiver limiting filter unit (415), the control room transceiver zero-intermediate frequency conversion part unit (414), the control room analog-to-digital conversion unit (412) and the control room integrated service processing unit (411) to convert the wireless signals into audio signals for transmission to the sending and receiving device.

18. The noise reduction communication system according to claim10, 11 or 12 is **characterized in that** both the patient transceiver and the control room transceiver are provided with an antenna configured and modulated for a transmitting circuit. The antenna is for a directional narrow transmission channel. A narrow channel (51E) is provided between the patient transceiver and the control room transceiver and used for signal transmission and exchange of the antenna.

19. The noise reduction communication system according to claim 18 is **characterized in that** the narrow channel (51E) is located outside the scanning area.

20. The noise reduction communication system according to claim 1 is **characterized in that** the patient transceiver is provided with a wireless transmitting airbag alarm device which has an airbag ball (1F) incorporated therein. The patient transceiver is further provided with a pneumatic switch (4F) and a control alarm circuit (8F), wherein one end of the control alarm circuit (8F) is connected with the pneumatic switch (4F); the other end of the pneumatic switch (4F) is connected with the air tube through a connector (2F), the other end of the air tube is connected with the airbag ball, and the other end of the control alarm circuit is connected with the integrated service processing unit of the patient transceiver.

21. A noise reduction communication system according to claim 14 is **characterized in that** the second sending channel comprises a shielding box (22F) and a shielding box (32F) , wherein the shielding box (32F) is provided with a microphone (28F) having an amplifier (27F), an amplifying circuit (29F) and a speaker (30F) with an acoustic wave concentrator (31F) which are successively connected, wherein a speaker (30F) is connected with an amplifying circuit (34F) through an air tube (33F). And a noise reduction module (36F), an amplifying circuit (23F) and a speaker (24F) with an acoustic wave concentrator (25F) are successively connected in the shielding box (22F), wherein the small end of the acoustic wave concentrator (25F) is connected with the small end of the amplifier (27F) through an air tube, and the other end of the noise reduction module is connected with the small microphone (21F);
The first sending channel comprises a speaker (35F) and a integrated unit (34F) which are connected successively.

22. A noise reduction communication system according to claim 14 is **characterized in that** the second sending channel comprises a shielding box (12F) which has a noise reduction module (11F), an amplifying circuit (13F) and a speaker (14F) with an acoustic wave concentrator (15F) successively incorporated therein. The small end of the acoustic wave concentrator (25F) is connected with the small end of an amplifier (17F) located outside of the shielding box through an air tube. The big end of the amplifier (17F) is connected with a microphone (18F), the other end of the noise reduction module is linked to a small microphone (10F), and the other end of the microphone (18F) is connected with a filter (19F);
The first sending channel comprises a speaker (21F), a integrated unit (20F) and the filter (19F) which are connected successively.

23. The noise reduction communication system according to claim 1, 2 or 3 is **characterized in that** the air tube microphone headset has no metal material from the bow to the air tube socket.

24. The noise reduction communication system according to claim 14 is **characterized in that** the additional airbag alarm is provided for patients who can not speak. A pneumatic sound producer (36A) is installed on the sound receiving surface of the sound collector (4D) in the air tube microphone headset and covered by a cover (34A) which is flexibly installed on the sound collector (4D). The cover (34A) can be opened to remove the pneumatic sound producer (36A) and the airbag (38A) when the pneumatic sound producer (36A) is not needed.
If a patient needs to contact the doctor in the control room, press the airbag (38A) several times and the air pressure of the airbag (38A) will be transmitted to the pneumatic sound producer (36A) through the passage of the air tube (37A). The pneumatic sound producer (36A) will then give an alarm sound, which will be transmitted to the sound collector (4D) and the air tube microphone headset and then through the patient transceiver or the acoustic-electro conversion device and finally be delivered to the control room.
